# EUROPÄISCHE PATENTSCHRIFT

(11) **EP 1 930 832 B1**
(45) Veröffentlichungstag und Bekanntmachung des Hinweises auf die Patenterteilung: **23.11.2016**
(21) Anmeldenummer: 06025315.0
(22) Anmeldetag: 07.12.2006
(51) Int. Cl.: G06F 19/00

(54) **Automatische Landmarkenbestimmung anatomischer Strukturen**
Automatic landmark determination of anatomic structures
Détermination automatique de points de repère de structures anatomiques

(43) Veröffentlichungstag der Anmeldung: 11.06.2008
(73) Patentinhaber: Brainlab AG, 85622 Feldkirchen (DE)
(72) Erfinder: Witte, Jens, 80339 München (DE)
(74) Vertreter: Schwabe - Sandmair - Marx

(56) Entgegenhaltungen:
- US-A- 5 623 586
- CUISENAIRE, O. ; THIRAN, J. ; MACQ, B. ; MICHEL, C. ; DE VOLDER, A.: "Automatic registration of 3D MR images with a computerized brain atlas" SPIE MEDICAL IMAGING, Bd. 2710, 1996, Seiten 438-448, XP002430061

## Beschreibung

Die vorliegende Erfindung betrifft die automatische Landmarkenbestimmung anatomischer Strukturen, eine entsprechende Einrichtung und ein Verfahren. Das Verfahren ist insbesondere Teil eines vorzugsweise Software basierten chirurgischen Planungsverfahren, das diese automatische Landmarkenbestimmung zur Beurteilung von anatomischen Strukturen verwendet.

Gemäß dem Stand der Technik wird chirurgische Planungssoftware angeboten, die die Planung eines chirurgischen Eingriffs zur Änderung einer anatomischen Struktur unterstützen. Beispiele lauten wie folgt:
Onyx Ceph 2.5 Sofware, http://www.onyx-ceph.de/
FYI Technologies, Dr. Ceph., http://www.fyitek.com/software.htm
ElleSoft.com, Lightning Ceph
αHAL Software Viewbox 3.1, http://www.dhal.com/index.htm
SimPlant CMF, http://www.materialise.com/simplant/cmf_ENG.html

Die oben genannten Softwarebeispiele betreffen den Schädelknochen, insbesondere Kiefer- und Gesichtsknochen. Bei dieser chirurgischen Planungssoftware werden anatomische Landmarken, die für die jeweilige anatomische Struktur typische Positionen bzw. Bereiche darstellen, wie beispielsweise eine Vertiefung bzw. eine Öffnung oder ein Fortsatz, vom Bediener (Chirurgen) eingegeben. Anatomische Landmarken sind insbesondere prägnante Ausbildungen einer knöchernen oder Weichteil-(Haut) Oberfläche, wie Spitzen, Kanten, Öffnungen und Vertiefungen, die zum Beispiel für Muskelansätze, Gefäßkanäle, Nervenkanäle usw. in der anatomischen Struktur vorgesehen sind und die insbesondere durch Computertomographenaufnahmen (CT), Kernspintomographenaufnahmen (MRT), Röntgenaufnahmen (X-Ray) oder andere diagnostische Bildgebungsverfahren darstellbar sind. Die Eingabe der anatomischen Landmarken erfolgt in den angegebenen Softwarebeispielen manuell, wobei die eingegebenen anatomischen Landmarken i in den diagnostischen Daten der anatomischen Struktur des Patienten von der Planungssoftware gezeigt werden. Die Software stellt die anatomischer Strukturen in zweidimensionalen oder dreidimensionalen Bildern (Modellen) dar. In diesen zweidimensionalen oder dreidimensionalen Bildern wird die Position der Landmarken mit Hilfe der Eingabe des Bedieners definiert. Mittels einer Vermessungssoftware, beispielsweise mittels digitaler Schablonen wird dann die räumliche Beziehung dieser Landmarken untereinander (Abstände, Winkel, gemeinsame Ebenen) von dem Bediener beurteilt, wobei hierbei beispielsweise auf allgemeine Harmoniestandards (Schönheitschirurgie) oder auf sonstige als ideal angesehene räumliche Beziehungen (beispielsweise zwischen Hüftpfanne und Oberschenkelkopf oder Oberkiefer und Unterkiefer) zurückgegriffen wird.

Cuisenaire, O.; Thiran, J.; Macq, B.; Michel, C.; De Volder, A.: "Automatic registration of 3D MR images with a computerized brain atlas" SPIE MEDICAL IMAGING, Bd. 2710, 1996, Seiten 438-448, XP002430061 offenbart eine Transformation von MR-Bildern auf einen Atlas (Computerized Brain Atlas), um Strukturen wie Sulci und Gyri zu identifizieren und zu labelln. Hierzu werden mathematisch vereinfachte Oberflächentopologien im MR-Bild bestimmt. Abstände zwischen Punkten auf diesen Oberflächentopologien werden als Optimierungskriterium zur Bestimmung der Transformation herangezogen.

US-A-5272760 offenbart ein Gerät zur Bestimmung von Abständen zwischen anatomischen Merkmalen, die in einem Röntgenbild gezeigt sind.

Aufgabe der Erfindung ist es die Bestimmung anatomischer Landmarken, insbesondere die Planung chirurgischer Eingriffe zu erleichtern bzw. zu beschleunigen sowie automatische Auswertungsergebnisse bereitzustellen.

Vorstehende Aufgabe wird durch die Gegenstände der unabhängigen Ansprüche gelöst. Vorteilhafte Weiterbildungen gehen aus den abhängigen Ansprüchen hervor.

Die erfindungsgemäßen Gegenstände erlauben es insbesondere die Zeit zu verkürzen, die ein Bediener (Chirurg) benötigt, um chirurgische Eingriffe, zum Beispiel am Schädel oder Kieferknochen durchzuführen. Außerdem unterstützen sie die Analyse des menschlichen Skelettes eines Patienten oder der Weichgewebe-Anatomie, wie dies zum Planen eines chirurgischen Eingriffs vonnöten sein kann.

Eine erfindungsgemäße automatische Landmarken-Bestimmungseinrichtung umfasst vorzugsweise ein Musterspeichermittel, das zumindest ein anatomisches Musterbild und die zugehörigen anatomischen Landmarken speichert. Die Positionen der Landmarken in dem zweidimensionalen oder dreidimensionalen Bild sind also durch das Musterspeichermittel neben den Bilddaten, die eine anatomische Struktur darstellen, gespeichert. Vorzugsweise ist insbesondere bekannt, welche Bildpunkte des anatomischen Musterbildes mit anatomischen Landmarken übereinstimmen und insbesondere um welche anatomische Landmar
ken es sich dabei bei der jeweiligen Position handelt. Vorzugsweise gibt es also eine Zuordnungsvorschrift zwischen den Bilddaten, die die anatomische Struktur darstellen und anatomischen Landmarken. Die anatomischen Landmarken können dabei zum Beispiel zusammen mit dem anatomischen Musterbild als Bilddaten so abgespeichert sein, dass sie bei Aufrufen des anatomischen Musterbilds und bei dessen Darstellung mit dargestellt werden. Die anatomischen Landmarken können aber auch separat abgespeichert sein, zum Beispiel in einer Tabelle, die die Position der Landmarke in dem Musterbild und insbesondere die Art der anatomischen Landmarke angibt.

Die erfindungsgemäße Landmarken-Bestimmungseinrichtung umfasst weiter vorzugsweise ein Eingabemittel zum Eingeben eines anatomischen Patientenbildes bzw. Datensatzes, beispielsweise eines zweidimensionalen Bildes oder dreidimensionalen Bildes. Das anatomische Patientenbild zeigt vorzugsweise einen Bereich einer anatomischen Struktur des Patienten, beispielsweise Schädelknochen oder Kieferknochen oder Hüftknochen oder Oberschenkelknochen. Das anatomische Patientenbild ist insbesondere ein oder eine Reihe von Röntgenbildern, , CT-Bildern oder Kernspinbildern (MRT).

Erfindungsgemäß ist insbesondere ein Transformationsmittel vorgesehen, das insbesondere einen Abgleich (Matching) zwischen dem eingegebenen anatomischen Patientenbild (Patientendatensatz) und dem Musterbild (Musterdatensatz) vornimmt.

Das erfindungsgemäße Transformationsmittel ist ausgebildet, eine Bildanpassungs-Transformation durchzuführen, die das Musterbild in das Patientenbild überführt. Dabei handelt es sich insbesondere um eine stetige oder nahezu stetige Transformation, wie sie von Morphingalgorithmen oder "image fusion"-Algorithmen (Bild-Fusionsalgorithmen) verwendet werden. Die Bildanpassungs-Transformation wird also insbesondere dadurch definiert, dass sie das Musterbild und Patientenbild bestmöglich übereinander legt und/oder dass sie die stetige Umwandlung (insbesondere Veränderung oder Deformation) oder eine schrittweise Umwandlung (mit einer Vielzahl von Schritten) des Musterbildes in das Patientenbild leistet.

Auf die Landmarken des Musterbildes wird erfindungsgemäß die Bildanpassungs-Transformation angewendet, die das Musterbild in das Patientenbild überführt. Zum Beispiel werden die Koordinaten der Landmarken, die im Musterbild definiert sind , demselben Morphingalgorithmus unterzogen, wie die übrigen Bilddaten des Musterbildes. Das Musterbild stellt eine anatomische Struktur dar, die als anatomische Musterstruktur durch die Bildanpassungs-Transformation in die anatomische Struktur des Patienten übergeführt werden soll. Dabei werden die anatomischen Landmarken insbesondere der gleichen Transformation unterzogen, wie die Bilddaten, die sich bei der Position der anatomischen Landmarken befinden. Die Positionen der Landmarken ändern sich durch die Transformation also bevorzugt genau so wie die Positionen der Musterbilddaten, die sich vor der Transformation an derselben Position befanden, wie die Landmarken. Somit erhält man durch die Transformation die Positionen der Landmarken im Patientenbild. Sind die Positionen der Landmarken separat von den Musterbildern gespeichert, so werden die Positionen der Bildanpassungstransformation unterzogen, um so die Position der Landmarken im Patientenbild zu bestimmen.

Die transformierten Landmarken (bzw. die transformierten Positionen der Landmarken) werden also bevorzugt als Landmarken des Patientenbildes (bzw. als Positionen der Landmarken im Patientenbild) bestimmt bzw. als solche identifiziert. Es ist somit eine automatische Bestimmung der Landmarken für das Patientenbild erfolgt. Soweit hierin von einer "Bestimmung der Landmarken" gesprochen wird ist hiermit die "Bestimmung der Positionen der Landmarken im Patientenbild" und/oder "die Kennzeichnung bzw. Bestimmung von Bildpunkten im Patientenbild als Landmarken, die im Patientenbild bestimmte Positionen einnehmen" gemeint.

Die Patientenbilder können verschiedenste anatomische Strukturen eines menschlichen oder tierischen Körpers darstellen. Um für eine Vielzahl von anatomischen Strukturen und/oder für eine Vielzahl von Ansichten der anatomischen Strukturen die Landmarken ermitteln zu können, umfasst das Musterspeichermittel vorzugsweise eine Vielzahl anatomischer Musterbilder, die anatomische Strukturen darstellen. Die anatomischen Musterbilder können die zugeordneten Landmarken bereits enthalten, zum Beispiel als Bildpunkte und/oder Bilddaten oder Landmarken können separat gespeichert sein, die bestimmten Positionen in den anatomischen Musterbildern zugeordnet sind. Die beiden zuvor genannten Optionen sollen hierin durch den Begriff "anatomische Musterbilder mit zugeordneten Landmarken" umfasst sein.

Nach der Bestimmung der Landmarken des Patientenbildes erfolgt vorzugsweise ein Abspeichern des Patientenbildes mit zugeordneten bestimmten Landmarken. Diese Abspeichern kann ebenfalls entweder so erfolgen, dass die bestimmten Landmarken als Bilddaten im Patientenbild dargestellt werden und zusammen mit den übrigen Bilddaten des Patientenbild abgespeichert werden. Beispielsweise kann die bestimmte Landmarke durch einen schwarzen Punkt im Patientenbild dargestellt werden. Insbesondere kann eine Beschriftung neben dem (schwarzen) Punkt ebenfalls als Bilddaten mit abgespeichert werden. Vorzugsweise erfolgt eine separate Abspeicherung der Landmarken mit der Position, die den Landmarken im Patientenbild zugeordnet ist. Auf diese Art und Weise lassen sich die Landmarken im Patientenbild je nach Wunsch des Bedieners einblenden oder ausblenden.

Um die Beurteilung einer anatomischen Struktur zu erleichtern und/oder die Planung eines chirurgischen Eingriffs zu erleichtern, umfasst die erfindungsgemäße Landmarken-Bestimmungseinrichtung vorzugsweise ein Mittel, um geometrische Größen zu bestimmen, die von der relativen Lage der bestimmten Landmarken zueinander abhängen und/oder diese relative Lage beschreiben. Beispiele für derartige geometrische Größen sind der Abstand zwischen zwei Landmarken, Winkel zwischen Geraden, die verschiedene Landmarken miteinander verbinden, die Lage einer Ebene, in der sich zwei oder mehr Landmarken befinden, das Verhältnis von Abständen zwischen verschiedenen Landmarken usw.

Vorzugsweise ist ein Vergleichsmittel vorgesehen, das die bestimmten geometrischen Größen mit Sollgrößen vergleicht. Hierzu ist vorzugsweise eine Soll-Größen-Datenbank vorgesehen, die übliche geometrische Größen und/oder geometrische Größen, die z.B. in der medizinischen Literatur als Standard angesehen werden, speichert. Diese Soll-Größen vergleicht vorzugsweise das Vergleichsmittel mit den bestimmten geometrischen Größen, die das Patientenbild betreffen und die anhand der bestimmten Landmarken bestimmt wurden. Vorzugsweise wird das Vergleichsergebnis angezeigt, z.B. als Angabe der prozentualen Abweichung der bestimmten geometrischen Größe von der entsprechenden Soll-Größe. Hierzu ist vorzugsweise ein Anzeigemittel, wie zum Beispiel ein Bildschirm vorgesehen.

Die erfindungsgemäße Landmarkenbestimmungseinrichtung umfasst insbesondere einen Computer, auf dem ein Programm läuft, das die vorgenannte Landmarken-Bestimmungseinrichtung in Zusammenwirkung mit dem Computer realisiert.

Auch ist die Erfindung auf ein entsprechendes Landmarken-Bestimmungsverfahren gerichtet, wobei das vorgenannte Programm dieses Verfahren implementiert. Ebenfalls ist die Erfindung auf ein chirurgisches Planungsverfahren sowie ein entsprechendes Programm gerichtet, das das Landmarken-Bestimmungsverfahren einsetzt.

Bei der folgenden detaillierten Beschreibung werden weitere Merkmale und Vorteile der Erfindung offenbart, die miteinander kombiniert werden können.
Figur 1 zeigt ein Beispiel für ein dreidimensionales Patientenbild;
Figur 2 zeigt ein dreidimensionales anatomisches Musterbild mit Landmarken;
Figur 3a und Figur 3b zeigen zweidimensionale Musterbilder;
Figur 4 zeigt ein Patientenbild mit bestimmten Landmarken;
Figur 5 und Figur 6 zeigen schematisch die Bestimmung geometrischer Größen;
Figur 7 zeigt den schematischen Aufbau einer erfindungsgemäßen automatischen Landmarken-Bestimmungseinrichtung.

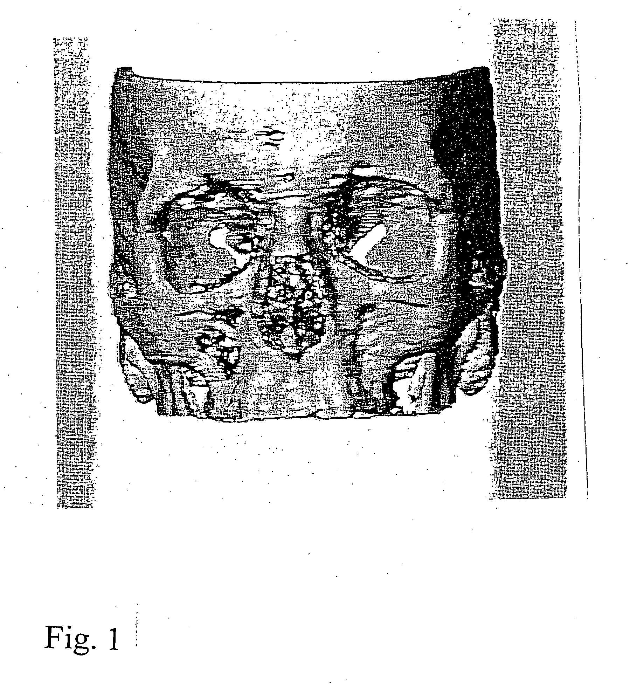

Figur 1 zeigt ein Beispiel für ein dreidimensionales Patientenbild, generiert aus einem zweidimensionalen diagnostischen Patientendatensatz, der beispielsweise über Computertomographie (CT) oder über Kernspinresonanztomographie (MRT) bestimmt wurde. Diese Daten wurden beispielsweise mit Hilfe eines erfindungsgemäßen Softwareprogramms, also beispielsweise eines Programms zum Planen chirurgischer Eingriffe (chirurgische Planungssoftware) in einen Computer geladen. Das in Figur 1 gezeigte Patientenbild zeigt keine Landmarken. Diese wären Herkömmlicherweise von einem Bediener (z.B. Arzt), beispielsweise durch Betätigen einer Maus anhand des Bildes zu identifizieren. Wie bereits oben ausgeführt, wird dies erfindungsgemäß jedoch automatisch durchgeführt.

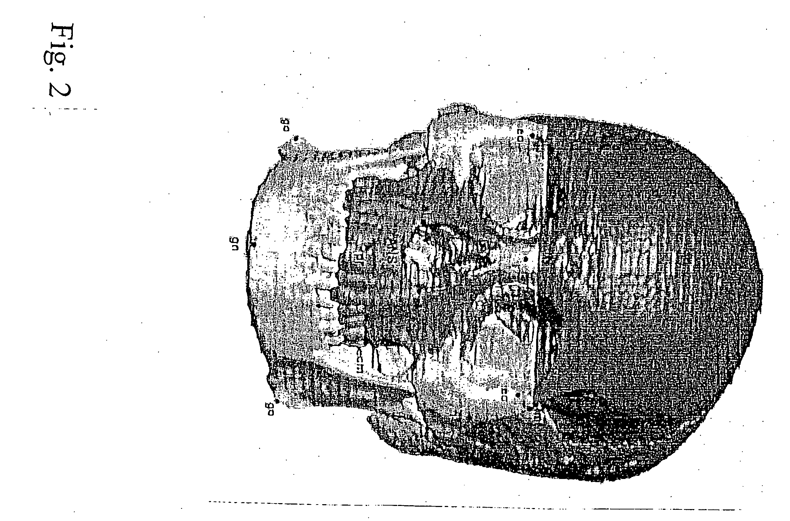

Hierzu wird auf ein anatomisches Musterbild bzw. Musterdatensatz zurückgegriffen, wie es beispielsweise in Figur 2 zu sehen ist. Das anatomische Musterbild, hier ein dreidimensionales Modell aus einem digitalen anatomischen Atlas, wird von der erfindungsgemäßen Einrichtung verarbeitet. Wie in Figur 2 zu sehen, sind neben der Abbildung eines menschlichen Schädels auch Landmarken zu sehen, die mit schwarzen Punkten gezeichnet wurden und Abkürzungen tragen wie N, pr, ANS, go, gn usw. Diese anatomischen Landmarken fehlen noch in dem Figur 1 gezeigten Schädelbild. Die erfindungsgemäße Landmarken-Bestimmungseinrichtung oder das erfindungsgemäße Programm lädt vorzugsweise die in Figur 2 gezeigten Daten zum Beispiel in den Programmhintergrund, um einen Ausgangspunkt zur Bestimmung der Landmarken zu bilden. Durch das erfindungsgemäße Transformationsmittel, beispielsweise ein Morphingalgorithmus oder einen Algorithmus zur Bildfusion (image fusion") werden dann die in Figur 2 gezeigten Bilddaten (Musterbilddaten) in die in Figur 1 gezeigten Patientendaten überführt. Dabei durchlaufen die in Figur 2 gezeigten anatomischen Landmarken dieselbe Transformation, wie die in Figur 2 gezeigten Bilddaten, die den Schädel darstellen. Genauer durchlaufen die Landmarken diejenige Transformation, die die Bilddaten durchlaufen, die sich an der Position der Landmarken befinden.

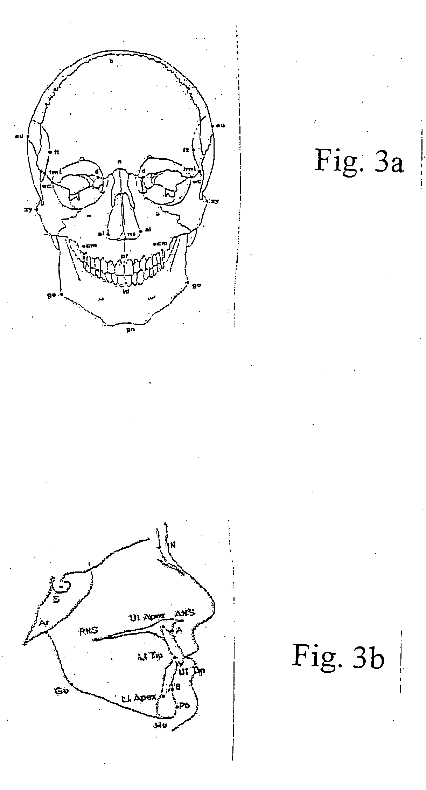

Figur 3 zeigt zweidimensionale Musterbilder mit Landmarken, die als schwarze Punkte dargestellt sind, wobei neben den Punkten Abkürzungen angegeben sind, die die Landmarken bezeichnen. Die Abkürzungen lauten entsprechend dem in der Medizin etablierten Bezeichnungsstandard (beispielsweise al, ns, ld, pr, n, ...).

Ist durch die erfindungsgemäße Bildanpassungs-Transformation das Musterbild in das Patientenbild übergeführt, so ergeben sich hieraus die anatomischen Landmarken des Patientenbildes. Die Bildanpassungs-Transformation kann insbesondere so gestaltet werden, dass die übergeführten Daten des Musterbildes zumindest mit einer vorbestimmten Genauigkeit mit den Patientenbilddaten übereinstimmen. Für den Algorithmus kann hierzu ein Ähnlichkeitskriterium festgelegt werden, welches als Abbruch bzw. Zielkriterum genutzt wird.

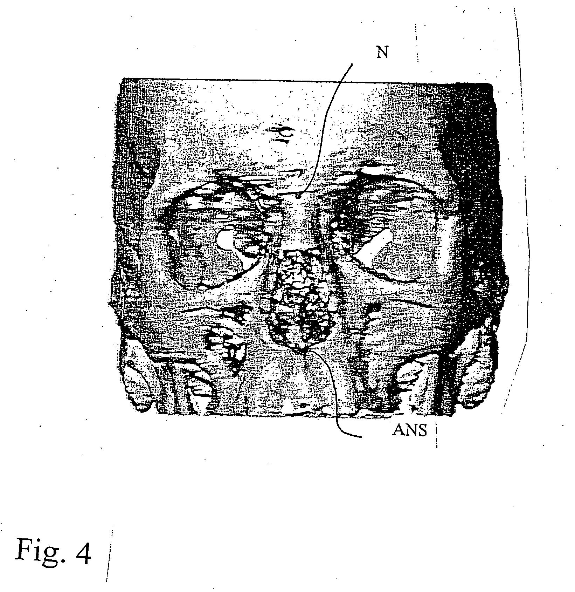

Figur 4 ist ein Beispiel für ein Patientenbild mit einzelnen, punktförmig dargestellten Landmarken. Zwei der Landmarken, nämlich die Landmarken N und ANS sind in Figur 4 hervorgehoben. Sie ergeben sich automatisch durch die Bildanpassungs-Transformation, die die in Figur 2 gezeigten Landmarken in das in Figur 1 gezeigte Bild überführen.

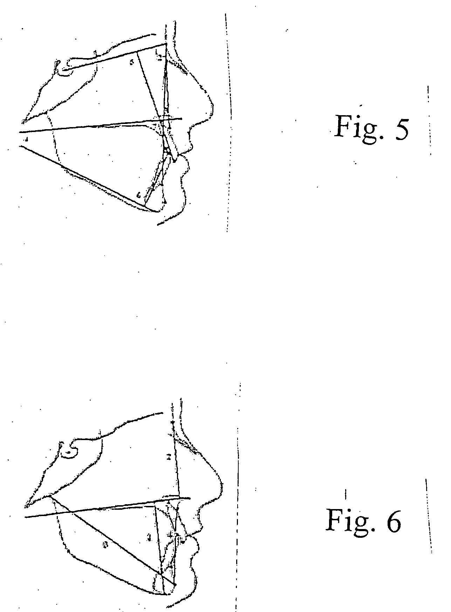

Figur 5 und Figur 6 zeigt die Definition anatomischer Größen, wobei die mit den Ziffern 1, 2, 3, 4, 5 und 6 bezeichneten Linien verschiedene Landmarken verbinden. Die Länge dieser Linien, also der Abstand zwischen den Landmarken stellen ein Beispiel für geometrische Größen dar. Entsprechendes gilt für die Winkel zwischen den Linien, diese stellen ebenfalls ein Beispiel für geometrische Größen dar. Diese geometrischen Größen werden vorzugsweise mit Soll-Größen verglichen, die zum Beispiel in einer Datenbank abgespeichert sind und die den allgemeinen Standards der medizinischen Literatur entsprechen. Ein Operateur kann somit erkennen, inwieweit der Patient den Standards entspricht und inwieweit Abweichungen vorliegen. Insbesondere kann der Chirurg überprüfen, inwieweit zum Beispiel bei einer Kieferoperation chirurgische Eingriffe zu Abweichungen von den Soll-Größen führen und/oder die geometrischen Größen des Patienten den Soll-Größen durch eine Operation angenähert werden können (zum Beispiel bei einer Korrektur eines Überbisses).

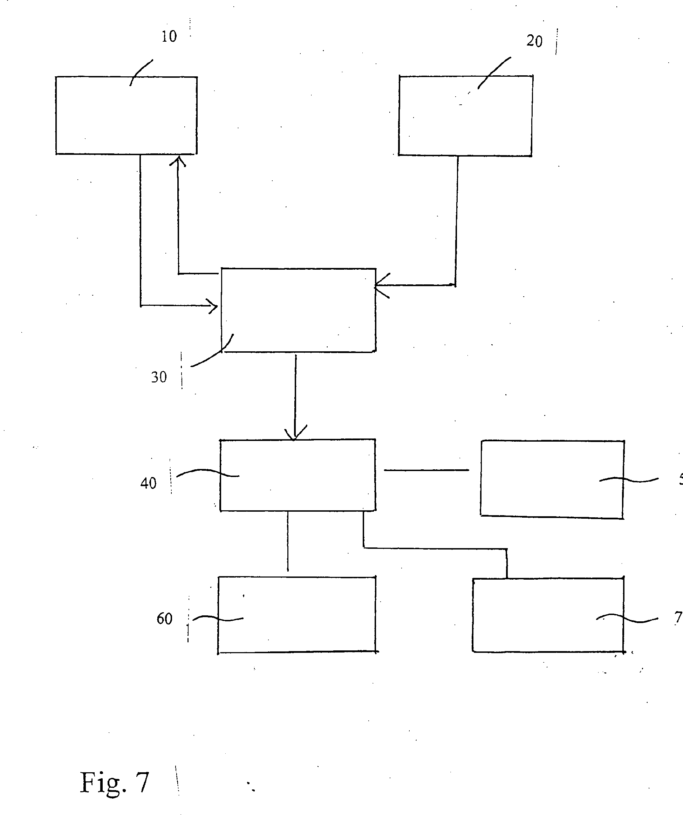

Figur 7 zeigt schematisch den Aufbau einer automatischen Landmarken-Bestimmungseinrichtung. Ein Musterspeichermittel 10, das beispielsweise als Festplatte in einem Computer ausgebildet ist, speichert einen digitalen anatomischen Atlas. Der Atlas umfasst Bilder, denen anatomischen Landmarken zugeordnet sind. Ein Eingabemittel 20 erlaubt die Eingabe anatomischer Patientenbilder. Beispielsweise ist das Eingabemittel 20 als Netzwerkschnittstelle ausgebildet, über die Daten aus einem diagnostischen Bildgebungsverfahren eingelesen werden können. Eine andere beispielhafte Ausführungsform ist die Ausbildung des Eingabemittels als Daten-Laufwerk. Die Daten von dem Musterspeichermittel und von dem Eingabemittel werden dem Transformationsmittel 30 zugeführt, der die Bildanpassungs-Transformation am anatomischen Musterbild so durchführt, dass das anatomische Musterbild in das Patientenbild übergeführt wird. Vorzugsweise umfasst das Transformationsmittel 30 ein Auswahlmittel, um anhand des Patientenbildes aus der anatomischen Datenbank 10 ein anatomisches Musterbild auszuwählen, das dem Patientenbild am ähnlichsten ist. Hierzu wird beispielsweise ein Datenbanksuchalgorithmus verwendet, der einen Mustererkennungsalgorithmus einsetzt ("pattern recognition algorithm"). Vorzugsweise wird also von dem Musterspeichermittel 10 dasjenige anatomische Musterbild dem Transformationsmittel 30 zugeführt, das dieses anhand des Patientenbildes ausgewählt hat. Sind von dem Transformationsmittel 30 nun die Landmarken bestimmt worden, so werden diese vorzugsweise dem Größenbestimmungsmittel 40 zugeführt. Das Größenbestimmungsmittel 40 bestimmt die geometrischen Größen, die von der relativen Lage der bestimmten Landmarken abhängen. Vorzugsweise umfasst das Größenbestimmungsmittel ein Vergleichsmittel, das auf eine Soll-Größen-Datenbank 50 zurückgreift, um die bestimmten geometrischen Größen mit Soll-Größen zu vergleichen. Das Vergleichsergebnis wird dann von dem Größenbestimmungsmittel 40 vorzugsweise einem Anzeigemittel 60 (z.B. Bildschirm) zugeführt, das neben diesem Vergleichsergebnis zusätzlich oder alternativ die Position der Landmarken, insbesondere in Verbindung mit den Patientenbilddaten anzeigt. Alternativ oder zusätzlich können die bestimmten geometrischen Größen in einem Speichermittel 70 abgespeichert werden. Im Speichermittel 70 können auch zusätzlich oder alternativ das Patientenbild, insbesondere mit den bestimmten Landmarken gespeichert werden.

## Patentansprüche

1. Automatische Landmarken-Bestimmungseinrichtung
mit einem Musterspeichermittel (10), das zumindest ein anatomisches Musterbild (Fig. 2) und Positionen anatomischer Landmarken im Musterbild speichert,
mit einem Eingabemittel (20) zum Eingeben eines anatomischen Patientenbildes (Fig. 1);
mit einem Transformationsmittel (30) zum Bestimmen der Positionen der Landmarken im Patientenbild, indem das Transformationsmittel (30) eine Bildanpassungs-Transformation auf die gespeicherten Landmarken des Musterbildes (Fig. 2) anwendet, wobei die Bildanpassungs-Transformation ausgebildet ist, das Musterbild (Fig. 2) in das Patientenbild (Fig. 1, Fig. 3) überzuführen;
mit einem Größenbestimmungsmittel (40), das geometrische Größen, die von der relativen Lage der mittels der Bildanpassungs-Transformation bestimmten Positionen der Landmarken abhängen, bestimmt;
mit einem Vergleichsmittel (40), das die bestimmten geometrischen Größen mit Soll-Größen vergleicht, die in einer Soll-Größen-Datenbank (50) abgespeichert sind; und
mit einem Anzeigemittel (60), das das Vergleichsergebnis anzeigt.

2. Automatische Landmarken-Bestimmungseinrichtung nach dem vorhergehenden Anspruch, bei welcher das Musterspeichermittel (20) einen anatomischen Atlas speichert, der eine Vielzahl von anatomischen Musterbildern (Fig. 2) mit zugeordneten Landmarken umfasst.

3. Anatomische Landmarken-Bestimmungseinrichtung nach einem der vorhergehenden Ansprüche, bei welchem die Bildanpassungs-Transformation einen Bild-Fusionsalgorithmus oder einen Morphinalgorithmus verwendet.

4. Automatische Landmarken-Bestimmungseinrichtung nach einem der vorhergehenden Ansprüche, bei welcher ein Speichermittel (70) die bestimmten Landmarken zusammen mit den Patientenbild (Fig. 3) abspeichert.

5. Anatomische Landmarken-Bestimmungseinrichtung nach einem der vorhergehenden Ansprüche, bei welcher das Transformationsmittel die Bilddaten des Musterbildes (Fig. 2) zusammen mit den Landmarken transformiert, um ein neues Patientenbild (Fig. 3) zusammen mit den Landmarken zu erhalten oder bei welchem das Transformationsmittel die Landmarken mit der Bildanpassungs-Transformation unabhängig von den Bilddaten des Musterbildes transformiert.

6. Verfahren zur Analyse von Patientenbildern, bei welchem
zumindest ein anatomisches Musterbild (Fig. 2) mit seinen anatomischen Landmarken bereitgestellt wird;
die Daten eines anatomischen Patientenbildes (Fig. 1) eingegeben werden;
eine Bildanpassungs-Transformation zum Bestimmen der Landmarker im Patientenbild, indem die Bildanpassungs-Transformation auf die Landmarken des anatomischen Musterbildes angewendet wird, wobei die Bildanpassungs-Transformation so ausgebildet ist, dass sie das anatomische Musterbild (Fig. 2) in das Patientenbild (Fig. 1, Fig. 3) überführt;
geometrische Größen, die von der relativen Lage der bestimmten Landmarken abhängen, bestimmt werden;
die bestimmten geometrischen Größen mit Soll-Größen verglichen werden; und
das Vergleichsergebnis angezeigt wird.

7. Verfahren zur Planung eines chirurgischen Eingriffes, bei welchem das Verfahren nach Anspruch 6 verwendet wird, um Landmarken in einem Patientenbild zu bestimmen und bei welchem die bestimmten Landmarken verwendet werden, um ein chirurgisches Verfahren zu planen.

8. Programm, das wenn es in einem Computer gespeichert wird oder wenn es in einem Computer geladen wird, das Verfahren nach Anspruch 6 oder 7 durchführt.

## Claims

1. An automatic landmark determining device, comprising:
a sample storage means (10) storing at least one anatomical sample image (Fig. 2) and positions of anatomical landmarks in the sample image,
an input means (20) for inputting an anatomical patient image (Fig. 1);
a transformation means (30) for determining the positions of the landmarks in the patient image by said transformation means (30) applying an image matching transformation to the stored landmarks of the sample image (Fig. 2), wherein said image matching transformation is configured to convert the sample image (Fig. 2) to the patient image (Fig. 1, Fig. 3 );
a value determination means (40) determining geometrical values which depend on a relative location of the positions of the landmarks determined by means of said image matching transformation;
a comparison means (40) comparing said geometrical values with nominal values stored in a nominal value database (50); and
a displaying means (60) displaying the comparison result.

2. The automatic landmarks determining device according to the preceding claim, wherein said sample storage means (20) stores an anatomical atlas comprising a plurality of anatomical sample images (Fig. 2) with associated landmarks.

3. The automatic landmark determining device according to one of the preceding claims, wherein said image matching transformation uses an image fusion algorithm or a morphing algorithm.

4. The automatic landmark determining device according to one of the preceding claims, wherein a storage means (70) stores the determined landmarks together with the patient image (Fig. 3).

5. The automatic landmark determining device according to one of the preceding claims, wherein said transformation means transforms the image data of the sample image (Fig. 2) together with the landmarks in order to obtain a new patient image (Fig. 3) together with the landmarks or wherein said transformation means transforms the landmarks using said image matching transformation independently of the image data of the sample image.

6. A method of analysing patient images, wherein
at least one anatomical sample image (Fig. 2) with associated anatomical landmarks is provided;
the data of an anatomical patient image (Fig. 1) are input;
an image matching transformation for determining the landmarks in the patient image by applying said image matching transformation to the landmarks of the anatomical sample image, wherein said image matching transformation is configured to convert the sample image (Fig. 2) to the patient image (Fig. 1, Fig. 3);
geometrical values which depend on a relative location of the determined landmarks are determined;
the determined geometrical values are compared to nominal values; and
the comparison result is displayed.

7. A method of planning a surgical procedure, wherein the method according to claim 7 is used to determine landmarks in a patient image, and wherein the determined landmarks are used to plan a surgical procedure.

8. A program which, when stored in a computer or when loaded in a computer, performs the method according to claim 6 or 7.

## Revendications

1. Dispositif de détermination automatique de points de repère
avec un moyen de stockage de modèles (10) qui stocke au moins une image anatomique modèle (fig. 2) et des positions de points de repère anatomiques dans l'image modèle,
avec un moyen d'entrée (20) pour entrer une image anatomique d'un patient (fig. 1);
avec un moyen de transformation (30) pour déterminer les positions des points de repère dans l'image du patient, le moyen de transformation (30) appliquant aux points de repère de l'image modèle (fig. 2) une transformation par adaptation de l'image, la transformation par adaptation de l'image étant conçue pour transférer l'image modèle (fig. 2) dans l'image du patient (fig. 1, fig. 3) ;
avec un moyen de détermination de dimensions (40) qui détermine des dimensions géométriques qui dépendent de la situation relative des positions des points de repère, déterminées au moyen de la transformation par adaptation d'image ;
avec un moyen de comparaison (40) qui compare les dimensions géométriques déterminées avec des dimensions nominales stockées dans une base de données de dimensions nominales (50) ; et
avec un moyen d'affichage (60) qui affiche le résultat de la comparaison.

2. Dispositif de détermination automatique de points de repère selon la revendication précédente, où le moyen de stockage de modèle (20) stocke un atlas anatomique qui comprend une pluralité d'images anatomiques modèles (fig. 2) avec des points de repère associés.

3. Dispositif de détermination automatique de points de repère selon l'une des revendications précédentes, où la transformation par adaptation de l'image utilise un algorithme de fusion d'images ou un algorithme de morphing.

4. Dispositif de détermination automatique de points de repère selon l'une des revendications précédentes, où un moyen de stockage (70) stocke les points de repère déterminés avec l'image de patient (fig. 3).

5. Dispositif de détermination automatique de points de repère selon l'une des revendications précédentes, où le moyen de transformation transforme les données d'image de l'image modèle (fig. 2) avec les points de repère pour obtenir une nouvelle image de patient (fig. 3) avec les points de repère, ou bien où le moyen de transformation transforme les points de repère au moyen de la transformation par adaptation de l'image, indépendamment des données d'image de l'image modèle.

6. Procédé pour l'analyse d'images de patient, où
au moins une image anatomique modèle (fig. 2) avec ses points de repère anatomiques est mise à disposition;
les données d'une image anatomique d'un patient (fig. 1) sont entrées ;
une transformation par adaptation de l'image est mise en oeuvre pour déterminer les points de repère dans l'image du patient, en appliquant la transformation par adaptation de l'image aux points de repère de l'image anatomique modèle, la transformation par adaptation de l'image étant réalisé de telle manière qu'elle transfère l'image anatomique modèle (fig. 2) dans l'image du patient (fig. 1, fig. 3) ;
des dimensions géométriques qui dépendent de la situation relative des points de repère déterminés sont déterminées ;
les dimensions géométriques déterminées sont comparées avec des dimensions nominales ; et
le résultat de la comparaison est affiché.

7. Procédé de planification d'une intervention chirurgicale utilisant le procédé selon la revendication 6 pour déterminer des points de repère dans une image d'un patient et utilisant les points de repère déterminés pour préparer une procédure chirurgicale.

8. Programme qui, lorsqu'il est enregistré sur un ordinateur ou chargé sur un ordinateur, exécute le procédé selon la revendication 6 ou 7.
